# EUROPÄISCHE PATENTSCHRIFT

(11) **EP 1 423 540 B1**
(45) Veröffentlichungstag und Bekanntmachung des Hinweises auf die Patenterteilung: **27.12.2006**
(21) Anmeldenummer: 02797605.9
(22) Anmeldetag: 21.08.2002
(51) Int. Cl.: C12Q 1/68, C12N 15/10, C12P 19/34

(54) **VERMEHRUNG VON RIBONUKLEINSÄUREN**
REPRODUCTION OF RIBONUCLEIC ACIDS
MULTIPLICATION D'ACIDES RIBONUCLEIQUES

(30) Priorität: 03.09.2001 DE 10143106
(43) Veröffentlichungstag der Anmeldung: 02.06.2004
(73) Patentinhaber: AmpTec GmbH, 22767 Hamburg (DE)
(72) Erfinder: KRUPP, Guido, 24622 Gnutz (DE); SCHEINERT, Peter, 22589 Hamburg (DE)
(74) Vertreter: UEXKÜLL & STOLBERG
(86) Internationale Anmeldenummer: PCT/EP2002/009348
(87) Internationale Veröffentlichungsnummer: WO 2003/020873

(56) Entgegenhaltungen:
- WO-A-00/75356
- WO-A-01/71036
- US-A- 5 514 545
- US-A- 5 665 547
- SAKKAR AND SOMMER: "ACCESS TO A MESSENGER RNA SEQUENCE OR ITS PROTEIN PRODUCT IS NOT LIMITED BY TISSUE OR SPECIES SPECIFITY" SCIENCE, AMERICAN ASSOCIATION FOR THE ADVANCEMENT OF SCIENCE,, US, Bd. 244, April 1989 (1989-04), Seiten 331-334, XP002144173 ISSN: 0036-8075

### SEQUENZPROTOKOLL

<110> Artus
<120> Vermehrung von Ribonukleinsäuren
<130> 58056
<140> Noch nicht vergeben
   <141> 2001-09-03
<160> 1
<170> PatentIn Ver. 2.1
<210> 1
   <211> 27
   <212> DNA
   <213> Künstliche Sequenz
<220>
   <223> Beschreibung der künstlichen Sequenz: Primer
<400> 1
   actaatacga ctcactatag gnnntct 27

## Beschreibung

Die vorliegende Erfindung betrifft Verfahren zur Vermehrung von Ribonukleinsäuren, bei denen man
(a) mittels eines Einzelstrang-Primers, einer RNA-abhängigen DNA-Polymerase und Desoxyribonukleotid-Monomeren einen DNA-Einzelstrang durch reverse Transkription aus einer RNA erzeugt;
(b) die RNA entfernt;
(c) mittels eines Einzelstrang-Primers, der die Sequenz eines Promotors und eine beliebige Sequenz von nicht mehr als 6 Nukleotiden umfaßt, einer DNA-Polymerase und Desoxyribonukleotid-Monomeren einen DNA-Doppelstrang erzeugt, wobei die Promotor-Sequenz die Bindung einer RNA-Polymerase ermöglicht und die Synthese eines RNA-Stranges initiiert;
(d) den Doppelstrang in Einzelstränge auftrennt;
(e) mittels eines Einzelstrang-Primers, der die Sequenz eines Promotors und eine beliebige Sequenz von nicht mehr als 6 Nukleotiden umfaßt, einer DNA-Polymerase und Desoxyribonukleotid-Monomeren DNA-Doppelstränge aus den in (d) entstandenen Einzelsträngen erzeugt, wobei die Promotor-Sequenz die Bindung einer RNA-Polymerase ermöglicht und die Synthese eines RNA-Stranges initiiert;
(f) mittels einer RNA-Polymerase und Ribonukleotid-Monomeren eine Vielzahl von RNA-Einzelsträngen erzeugt.

Die vorliegende Erfindung betrifft ferner Kits, welche für die Durchführung der erfindungsgemäßen Verfahren notwendigen Bestandteile umfassen.

Im Stand der Technik sind eine Vielzahl von Verfahren zur Vermehrung von Nukleinsäuren bekannt. Das bekannteste Verfahren ist die Polymerase-Ketten-Reaktion ("polymerase chain reaction" oder PCR), welche Mitte der 80er Jahre von Kary Mullis entwikkelt wurde (vgl. Saiki et al., Science, Vol. 230 (1985), 1350-1354; und EP 201 184).

Bei der PCR-Reaktion lagern sich Einzelstrang-Primer (Oligonukleotide mit einer Kettenlänge von üblicherweise 12 bis 24 Nukleotiden) an eine komplementäre, einzelsträngige DNA-Sequenz an. Die Primer werden mittels einer DNA-Polymerase und den Desoxyribonukleosidtriphosphaten (dNTPs, nämlich dATP, dCTP, dGTP, dTTP) zu einem Doppelstrang verlängert. Der Doppelstrang wird durch Hitzeeinwirkung in Einzelstränge aufgetrennt. Die Temperatur wird so weit gesenkt, dass sich erneut Einzelstrang-Primer an die DNA-Einzelstränge anlagern. Die Primer werden durch die DNA-Polymerase erneut zu einem Zweitstrang elongiert.

Bei Wiederholung der obigen Schritte ist eine exponentielle Vermehrung der Ausgangs-DNA-Stränge möglich, da die Reaktionsbedingungen so gewählt werden können, dass aus nahezu jedem DNA-Einzelstrang bei jedem Reaktionsdurchlauf ein Doppelstrang gebildet wird, der nachfolgend wieder in zwei Einzelstränge aufgespalten wird, welche wiederum als Matrize für weitere Stränge dienen.

Sofern vor diesem Verfahren eine reverse Transkription durchgeführt wird, bei der mittels einer RNA-abhängigen DNA-Polymerase ein DNA-Einzelstrang (die sogenannte cDNA) aus einer mRNA gebildet wird, kann die PCR-Reaktion auch unmittelbar auf die Vermehrung von Nukleinsäuren ausgehend von einer RNA-Sequenz anwendbar (vgl. EP 201 184).

Zu diesem Reaktions-Grundschema wurden in der Zwischenzeit eine Vielzahl von Alternativen entwickelt, die sich in Abhängigkeit des Ausgangsmaterials (RNA, DNA, Einzelstränge, Doppelstränge) und des Reaktionsproduktes (Vermehrung spezifischer RNA- oder DNA-Sequenzen in einer Probe oder Vermehrung aller Sequenzen) voneinander unterscheiden.

In den letzten Jahren werden zunehmend sogenannte Microarrays, zur Analyse von Nukleinsäuren verwendet. Dabei handelt es sich um Trägerplatten auf denen eine Vielzahl unterschiedlicher Nukleinsäure-Sequenzen (meist DNA) in verschiedenen Bereichen gebunden wurden. Üblicherweise wird in einem bestimmten sehr kleinen Bereich lediglich die DNA einer bestimmten Sequenz gebunden, wobei ein Microarray bis zu mehrere 1000 verschiedene Bereiche aufweisen und Sequenzen binden kann.

Werden diese Microarrays mit einer Vielzahl von verschiedenen Nukleinsäure-Sequenzen (meist ebenfalls DNA) aus einer zu untersuchenden Probe unter geeigneten Bedingungen (Salzgehalt, Temperatur, etc.) in Kontakt gebracht, so bilden sich komplementäre Hybride aus den in der Probe befindlichen und den auf der Platte gebundenen Sequenzen. Nicht-komplementäre Sequenzen können abgewaschen werden. Die Bereiche auf dem Microarray, welche DNA-Doppelstränge enthalten, werden ermittelt und ermöglichen einen Rückschluß auf die Sequenz und Menge der Nukleinsäure in der Ausgangsprobe.

Microarrays werden beispielsweise in entsprechenden Verfahren zur Analyse des Expressionsprofils von Zellen, also der Analyse der Gesamtheit der von bestimmten Zellen exprimierten mRNA-Sequenzen, eingesetzt (vgl. Lockhart et al., Nat. Biotechnol. 14 (1996), 1675-1680).

Da die Menge der für diese Analyse zur Verfügung stehenden mRNA üblicherweise beschränkt ist, sind speziell Verfahren zur Vermehrung von Ribonukleinsäuren entwickelt worden, die anschließend mittels Microarrays analysiert werden sollen. Dafür werden die Ribonukleinsäuren gegebenenfalls durch reverse Transkription in die stabilere cDNA-Form überführt.

Verfahren, die eine hohe Amplifikation einer RNA-Population einzelner Zellen ermöglichen sollen, werden beispielsweise in US 5,514,545 beschrieben. Bei diesem Verfahren wird ein Primer verwendet, der eine Oligo-dT-Sequenz und eine T7-Promotor-Region aufweist. Die Oligo-dT-Sequenz lagert sich an die 3'-Poly-A-Sequenz der mRNA an und initiiert so die reverse Transkription der mRNA. Nach alkalischer Denaturierung des RNA/DNA-Heteroduplex wird der zweite DNA-Strang unter Ausnutzung der Haarnadel-Struktur am 3'-Ende der cDNA als Primer gebildet und mittels Nuklease S1 zum linearen Doppelstrang geöffnet. Der DNA-Doppelstrang dient anschließend als Matrize für die T7-RNA-Polymerase. Die so erhaltene RNA dient wiederum als Primer für cDNA-Synthese, wobei hexamere Oligonukleotide beliebiger Sequenz (sog. "random primer") verwendet werden. Der zweite DNA-Strang wird nach Hitzedenaturierung unter Verwendung der genannten T-7-Oligo(dT)-Primer erzeugt. Diese DNA kann erneut als Matrize für die T7 RNA-Polymerase dienen.

Alternativ dazu offenbart US 5,545,522 dass auch ein einziges Primer-Oligonukleotid verwendet werden kann, um eine hohe Vermehrungsrate zu erzielen. Dafür wird cDNA aus einer RNA durch reverse Transkription erzeugt, wobei ein Primer verwendet wird, der folgdende Elemente aufweist: (a) 5'-dN₂₀, also beliebige Sequenz über eine Länge von 20 Nukleotiden; (b) minimaler T7-Promotor; (c) Transkriptionsstartsequenz GGGCG; und (d) Oligo-dT₁₅. Die Synthese des zweiten DNA-Stranges wird nach partiellem Verdau der RNA mittels RNase H durchgeführt, indem verbleibende RNA-Oligonukleotide als Primer für Polymerase I dienen. Die Enden der so erhaltenen DNA werden mit T4-DNA Polymerase geglättet.

Ein ähnliches Verfahren wird in US 5,932,451 offenbart, bei dem zusätzlich im 5'-proximalen Bereich noch zwei Box-Primersequenzen addiert werden, die ein doppeltes Immobilisieren via Biotin-Boxprimer ermöglichen.

In der Veröffentlichung von Sarkar und Sommer (Science, Vol. 244, 1989, S. 331-334) werden Verfahren zur Herstellung doppelsträngiger DNA ausgehend von RNA mit mindestens teilweise bekannter Sequenz offenbart. Die bekannte Sequenz ermöglicht die Verwendung von Primern, die neben der Promotersequenz einen Bereich umfassen, der zu der Zielsequenz komplementär ist.

Die genannten Verfahren zur Vermehrung von Ribonukleinsäuren weisen jedoch schwere Nachteile auf. So erzeugen die Verfahren Populationen von Ribonukleinsäuren, die in ihrer Zusammensetzung nicht der Ursprungs-Population entsprechen. Ein Grund dafür besteht in der Verwendung der T7-Promotor-Oligo-dT-Primer, welche primär Ribonukleinsäure-Sequenzen aus dem 3'-Bereich der mRNA amplifizieren. Ferner wurde experimentell belegt, dass die in den genannten Verfahren verwendeten, sehr langen Primer (mehr als 60 Nukleotide) die Bildung von Primer-Primer-Hybriden und eine unspezifische Amplifikation des Primers ermöglichen (Baugh et al., Nucleic Acids Res., 29 (2001). Die bekannten Verfahren erzeugen somit zu nicht unerheblichem Anteil Artefakte, die bei der weiteren Analyse der Nukleinsäuren stören.

Der vorliegenden Erfindung lag somit die Aufgabe zugrunde, ein Verfahren zur Vermehrung von Ribonukleinsäuren zur Verfügung zu stellen, das eine gleichmäßige Vermehrung der Ribonukleinsäuren im Ausgangsmaterial ermöglicht.

Diese Aufgabe wurde nunmehr durch Verfahren zur Vermehrung von Ribonukleinsäuren gelöst, bei denen man
(a) mittels eines Einzelstrang-Primers, einer RNA-abhängigen DNA-Polymerase und Desoxyribonukleotid-Monomeren einen DNA-Einzelstrang durch reverse Transkription aus einer RNA erzeugt;
(b) die RNA entfernt;
(c) mittels eines Einzelstrang-Primers, der die Sequenz eines Promotors und eine beliebige Sequenz von nicht mehr als 6 Nukleotiden umfaßt, einer DNA-Polymerase und Desoxyribonukleotid-Monomeren einen DNA-Doppelstrang erzeugt, wobei die Promotor-Sequenz die Bindung einer RNA-Polymerase ermöglicht und die Synthese eines RNA-Stranges initiiert;
(d) den Doppelstrang in Einzelstränge auftrennt;
(e) mittels eines Einzelstrang-Primers, der die Sequenz eines Promotors und eine beliebige Sequenz von nicht mehr als 6 Nukleotiden umfaßt, einer DNA-Polymerase und Desoxyribonukleotid-Monomeren DNA-Doppelstränge aus den in (d) entstandenen Einzelsträngen erzeugt, wobei die Promotor-Sequenz die Bindung einer RNA-Polymerase ermöglicht und die Synthese eines RNA-Stranges initiiert;
(f) mittels einer RNA-Polymerase und Ribonukleotid-Monomeren eine Vielzahl von RNA-Einzelsträngen erzeugt.

Erfindungsgemäß wurde überraschenderweise festgestellt, dass die genannte Kombination von Verfahrensschritten zu einer gleichmäßigen Amplifikation der im Ausgangsmaterial befindlichen Ribonukleinsäuren führt. Gleichzeitig verhindert das erfindungsgemäße Verfahren die Entstehung von Artefakten. Das erfindungsgemäße Verfahren stellt somit eine wesentliche Verbesserung der Verfahren zur Vermehrung von Ribonukleinsäuren dar und ermöglicht gleichzeitig eine Verbesserung der Verfahren zur Analyse von Ribonukleinsäuren mittels Microarrays.

Gemäß einer Ausführungsform des erfindungsgemäßen Verfahrens werden bei der Vermehrung RNA-Einzelstränge erzeugt, welche dieselbe Sinnrichtung (Sequenz) wie das RNA-Ausgangsmaterial aufweist. Alternativ dazu kann das erfindungsgemäßen Verfahren auch so durchgeführt werden, dass RNA-Einzelstränge beider Sinnrichtungen entstehen (Sequenz des Ausgangsmaterials und komplementäre Sequenz).

Der in (a) verwendete Einzelstrang-Primer umfaßt vorzugsweise eine Oligo-dT-Sequenz, also eine Sequenz, in der mehrere dT-Nukleotide aneiander gereiht sind. Dies weist den Vorteil auf, dass die Anlagerung des Primers im Bereich der poly-A-Sequenz der mRNA erfolgt. Es wird somit in der reversen Transkription nahezu ausschließlich mRNA transkribiert.

Erfindungsgemäß ist es besonders bevorzugt, das in (a) ein 5'-(dT)₁₈V-Primer für die reverse Transkription verwendet wird. Darunter wird ein Primer verstanden, der 18 dT-Desoxyribonukleotid-Monomere gefolgt von einem einzelnen Desoxyribonukleotid-Monomer anderer Art (also dA, dC oder dG, hier als V bezeichnet) aufweist. Dieser Primer ermöglicht eine reverse Transkription, die nahezu ausschließlich Sequenzen transkribiert, welche im unmittelbaren 5'-Bereich der Poly-A-Seqeunz beginnen. Die Verwendung dieses Primers unterdrückt somit die Bildung von Transkriptions-Artefakten, welche durch Anlagerung der üblichen Oligo-dT-Primer in größeren Poly-A-Bereichen der mRNA bei den im Stand der Technik bekannten Verfahren entstehen.

Erfindungsgemäß ist es ferner bevozugt, die RNA in den DNA-RNA-Hybriden in (b) durch RNase aufzuspalten. Dabei können beliebige RNasen verwendet werden. Die Verwendung von RNase I und/oder RNase H ist bevorzugt. Durch diesen Verfahrensschritt werden alle RNAs eliminiert, die beim ersten Schritt nicht in cDNA umgeschrieben wurden, darunter insbesondere ribosomale RNAs, aber auch andere zelluläre RNAs, die nicht den für mRNA charakteristischen Poly(A)-Schwanz aufweisen.

Die durch reverse Transkription entstandenen DNA-RNA-Hybride könnten alternativ auch durch Hitzeeinwirkung in die Einzelstrang-Form überführt werden. Die Verwendung der RNasen weist jedoch gegenüber der Hitzeeinwirkung den weiteren Vorteil auf, dass die ebenfalls in der Probe vorliegende genomische DNA nicht linearisiert wird und somit auch nicht als Hybridisierungspartner für die Primer in den nachfolgenden Schritten zur Verfügung steht. Besondere Vorteile des erfindungsgemäßen Verfahrens ergeben sich durch die Verwendung der RNase I, da dieses Enzym leicht durch Hitze inaktiviert werden kann. Das erfindungsgemäße Verfahren hat gerade die Vermehrung der Ribonukleinsäuren zum Ziel; eine stabile RNase könnte diesem Ziel entgegen wirken und müsste umständlich aus dem Reaktionsansatz entfernt werden.

In (c) wird ein Einzelstrang-Primer verwendet, der eine Promotor-Sequenz umfaßt. Eine Promotor-Sequenz ermöglicht die Bindung der RNA-Polymerase und initiiert die Synthese eines RNA-Stranges. Vorzugsweise verwendet man in (c) einen Einzelstrang-Primer, der die Sequenz eines hochspezifischen RNA-Polymerase-Promotors umfaßt, wie T7, T3, oder SP6.

Der Primer weist vorzugsweise eine Länge von nicht mehr als 35 Nukleotiden auf, wobei eine Länge von nicht mehr als 30 Nukleotiden besonders bevorzugt ist. Die Auswahl von Primern geeigneter Länge ist für das erfindungsgemäße Verfahren von besonderer Bedeutung, da im Stand der Technik häufig zu lange Primer (bis zu und mehr als 60 Nukleotide) verwendet wurden, welche zur Selbsthybridisierung neigen und in großem Umfang Artefakte erzeugen.

Gemäß einer Ausführungsform der Erfindung wird in (c) ein Einzelstrang-Primer verwendet, der neben der Sequenz des Promotors eine Sequenz von maximal 6, vorzugsweise 3 beliebigen Nukleotiden umfaßt. Diese Sequenz ermöglicht eine gleichmäßige Hybridisierung des Primers mit einem DNA-Strang beliebiger Seqeunz und somit auch die gleichmäßige Vermehrung aller DNA-Sequenzen in der Ausgangspopulation.

Erfindungsgemäß hat es sich als besonders vorteilhaft erwiesen, einen Primer zu verwenden, der neben dem Promotor eine besonders geeignete Sequenz von 6 Nukleotiden aufweist, nämlich die Sequenz: 5'-N-N-N-T-C-T-'3, worin N ein beliebiges Nukleotid darstellt (dATP, dCTP, dGTP oder dTTP).

Der in (c) verwendete Einzelstrang-Primer kann gemäß einer besonders bevorzugten Ausführungsform eine Länge von 27 Nukleotiden und folgende Sequenz aufweisen (SEQ ID NO.1, in dem Sequenzprotokoll als "<400> 1" bezeichnet):
5'-A-C-T-A-A-T-A-C-G-A-C-T-C-A-C-T-A-T-A-G-G-N-N-N-T-C-T-'3

In der SEQ ID NO. 1 stellt N wiederum ein beliebiges Nukleotid dar (dATP, dCTP, dGTP oder dTTP). Der Primer umfaßt die Sequenz des T7-RNA-Polymerase-Promotors. Der Transkriptionsstart der T7-RNA-Polymerase befindet sich in der als ⁺¹ dargestellten Position 5'-T-A-T-A-G⁺¹-G-N-N-N-3' der hier nur ausschnittsweise dargestellten obigen Sequenz.

Der Primer mit der oben genannten Sequenz (SEQ ID NO:1) ist ebenfalls Gegenstand der vorliegenden Erfindung.

Als DNA-Polymerase in (c) und (e) kann eine beliebige DNA-abhängige DNA-Polymerase verwendet werden. Vorzugsweise wird das Klenow-Fragment der DNA-Polymerase verwendet. Besondere Vorteile des erfindungsgemäßen Verfahrens ergeben sich, wenn man als DNA-Polymerase die Klenow-exo⁻ DNA-Polymerase verwendet. Für die DNA-Polymerisation in (a), (c) und (e) benötigt man ferner die Desoxyribonukleotid-Monomere, üblicherweise dATP, dCTP, dGTP und dTTP.

In (d) können die DNA-Doppelstränge durch beliebige Verfahren in Einzelstränge aufgetrennt werden. Vorzugsweise erfolgt die Auftrennung durch Hitzeeinwirkung.

Der in (e) eingesetzte Einzelstrang-Primer kann die identische Sequenz wie der in (c) eingesetzte Einzelstrang-Primer oder eine von diesem verschiedene Sequenz aufweisen. Es ist erfindungsgemäß bevorzugt, dass die in (c) und (e) eingesetzten Primer dieselbe Sequenz aufweisen.

Vor der Durchführung des Schrittes (f) kann es sinnvoll sein, überschüssige Primer und/oder Primer-verursachte Artefakte (z.B. Primer-Dimere) zu entfernen.

Die Bestimmung der in (f) eine eingesetzte RNA-Polymerase erfolgt in Abhängigkeit der in dem Primer vorhandenen Promotor-Sequenz. Wurde ein Primer mit Sequenzen der T7-Polymerase verwendet, so wird in (f) eine T7-RNA-Polymerase eingesetzt.

Für die Bildung von Ribonukleinsäuren in (f) benötigt man ferner Ribonukleotid-Monomere; üblicherweise werden ATP, CTP, GTP und UTP einsetzt.

Die erfindungsgemäßen Verfahren ermöglichen erstmals eine starke spezifische Vermehrung der RNA-Ausgangssequenzen, bei der die Gesamtsequenz der RNA-Population repräsentiert wird. Die Vermehrung der Ausgangs-RNA-Sequenz erfolgt vorzugsweise um einen Faktor von mindestens 500, wobei eine Vermehrung um einen Faktor von mindestens 3000 besonders bevorzugt ist.

Besondere Vorteile ergeben sich, wenn in
(a) ein 5'-(dT)₁₈V-Primer für die reverse Transkription verwendet wird; und in
(b) RNase zur Aufspaltung der DNA-RNA-Hybride verwendet wird; und in
(c) und (e) ein Primer mit der SEQ ID NO:1 und die Klenow-exo⁻ DNA-Polymerase verwendet werden; und in
(d) Hitzeeinwirkung zur Auftrennung der Doppelstränge verwendet wird; und in
(f) zunächst überschüssige Primer und/oder Primer-verursachte Artefakte entfernt und T7-RNA-Polymerase verwendet wird.

Eine weitere Vermehrung der Ribonukleinsäuren kann erzielt werden, wenn man ausgehend von den in (e) erzeugten DNA-Doppelsträngen mindestens einen PCR-Zyklus durchführt. Dafür werden die in (e) erzeugten DNA-Doppelstränge in Einzelstränge aufgetrennt und unter Verwendung von mindestens einem Einzelstrang-Primer, einer DNA-Polymerase und den Desoxyribonukleotid-Monomeren zu den DNA-Einzelsträngen jeweils komplementäre DNA-Stränge erzeugt. Die Auftrennung der DNA-Doppelstränge erfolgt vorzugsweise durch Hitzeeinwirkung. Eine weitere Vermehrung der Ribonukleinsäuren ist möglich, wenn mehrere PCR-Zyklen, vorzugsweise mindestens 2 oder 5 durchgeführt werden.

Dieses Vorgehen weist den besonderen Vorteil auf, dass in der nachfolgenden RNA-Polymerisation RNA-Moleküle beider Sinnrichtungen (Ursprungssequenz und komplementäre Sequenz) erzeugt werden.

Weitere Vorteile ergeben sich, wenn man für die Erzeugung der DNA-Doppelstränge (durch PCR) Einzelstrang-Primer verwendet, welche die Sequenz des in (c) und/oder (e) verwendeten Primers aufweist. Besonders bevorzugt werden Verfahren, bei denen man einen Einzelstrang-Primer der SEQ ID NO:1 verwendet.

Auch bei der Durchführung dieser Ausführungsform der vorliegenden Erfindung kann es vorteilhaft sein, den oder die Einzelstrang-Primer und Primer-verursachte Artefakte (z.B. Dimere) zu entfernen, bevor die RNA-Polymerase zugegeben wird.

Die vorliegende Erfindung betrifft ferner Kits, die alle Reagentien zur Vermehrung von Ribonukleinsäuren mittels des erfindungsgemäßen Verfahrens zur Verfügung stellen. Entsprechende Kits umfassen die folgenden Bestandteile:
(a) mindestens einen Einzelstrang-Primer, der die Sequenz eines Promotors und eine beliebige Sequenz von nicht mehr als 6 Nukleotiden umfaßt, wobei die Promotor-Sequenz die Bindung einer RNA-Polymerase ermöglicht und die Synthese eines RNA-Stranges initiiert;
(b) eine RNA-abhängige DNA-Polymerase;
(c) Desoxyribonukleotid-Monomere;
(d) eine DNA-abhängige DNA-Polymerase;
(e) eine RNA-Polymerase; und
(f) Ribonukleotid-Monomere.

Das Kit kann zwei oder mehrere verschiedene Einzelstrang-Primer umfassen, wobei es bevorzugt ist, dass ein Einzelstrang-Primer eine Oligo-dT-Sequenz umfaßt. Gemäß einer besonderen Ausführungsform umfaßt das Kit einen Einzelstrang-Primer mit einer 5'-(dT)₁₈V-Primer-Sequenz umfaßt, worin V Desoxyribonukleotid-Monomer bezeichnet, das nicht dT darstellt.

Das Kit kann zusätzlich RNase I und/oder RNase H und/oder einen Einzelstrang-Primer, der die Sequenz des T7-, T3- oder SP6-RNA-Polymerase-Promotors aufweist, umfassen. Der Einzelstrang-Primer weist neben der Sequenz des Promotors vorzugsweise eine beliebige Sequenz von maximal 6 Nukleotiden auf. Der Einzelstrang-Primer kann insbesondere die SEQ ID NO:1 aufweisen.

Die in dem Kit vorliegende DNA-Polymerase ist vorzugsweise das Klenow-Fragment der DNA-Polymerase, wobei die Verwendung der Klenow-exo⁻ DNA-Polymerase besonders bevorzugt ist.

Das Kit kann schließlich die T7-RNA-Polymerase, eine Zusammenstellung von Reagenzien zur Markierung und zum Nachweis von RNA und/oder DNA und einen oder mehrere Microarrays umfassen. Das Kit kann somit beispielsweise alle Bestandteile zur Durchführung einer Expressionsanalyse anbieten.

Erfindungsgemäß ist es besonders bevorzugt, daß das Kit, die folgenden Bestandteile umfasst:
(a) einen 5'-(dT)₁₈V-Primer für die reverse Transkription;
(b)RNase;
(c)einen Primer mit der in SEQ ID NO:1 gezeigten Seqeunz;
(d) Klenow-exo⁻ DNA-Polymerase;
(d)T7-RNA-Polymerase.

Üblicherweise werden die einzelnen Komponenten in getrennten Behältern verpackt sein; es kann jedoch auch möglich sein, Bestandteile, die in einem Verfahrensschritt verwendet werden gemeinsam in einen Behälter abzupacken.

Demgemäß betrifft die vorliegende Erfindung ferner Verfahren zur Analyse von Nukleinsäuren, bei dem man eine Ribonukleinsäure gewinnt, mittels eines der erfindungsgemäßen Verfahren vermehrt und unter Verwendung eines Microarrays analysiert. Die Ribonukleinsäure wird üblicherweise durch Isolierung aus einer biologischen Probe gewonnen. Die durch das erfindungsgemäße Verfahren vermehrte Ribonukleinsäure kann vor Analyse mittels Microarrays durch reverse Transkription in cDNA überführt werden. Das Verfahren ermöglicht die Analyse der Menge und/oder Sequenz der cDNA.

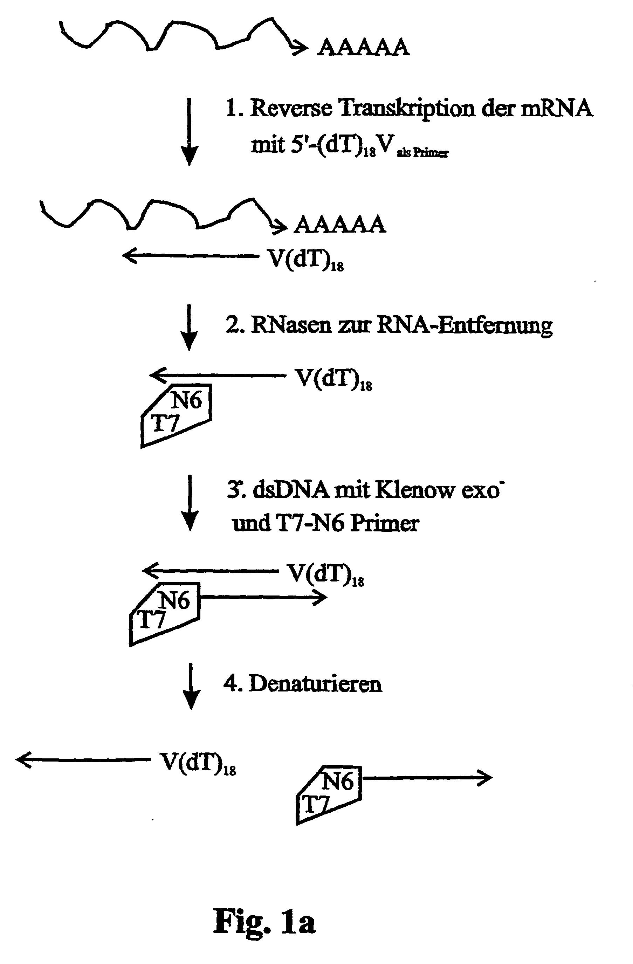

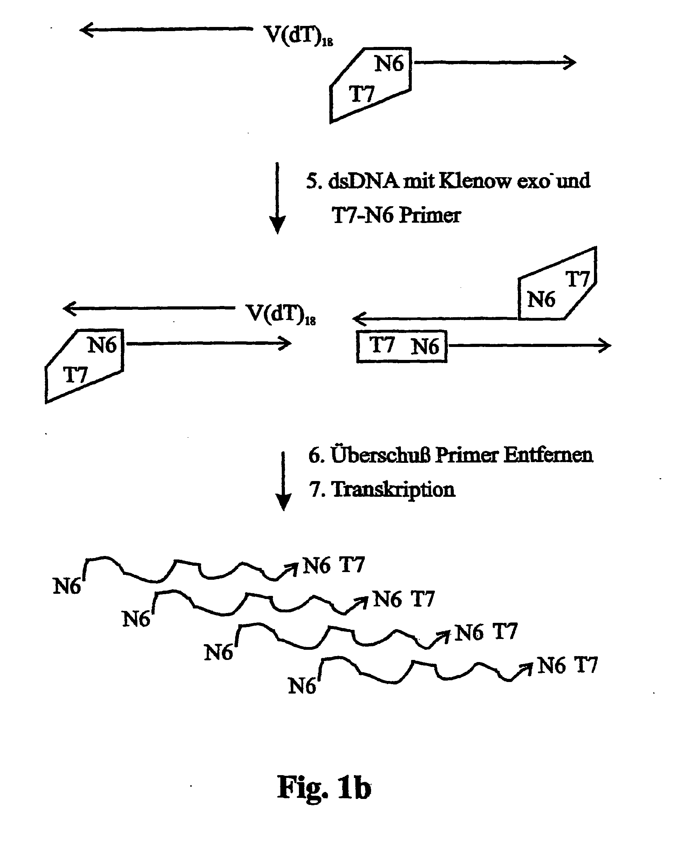

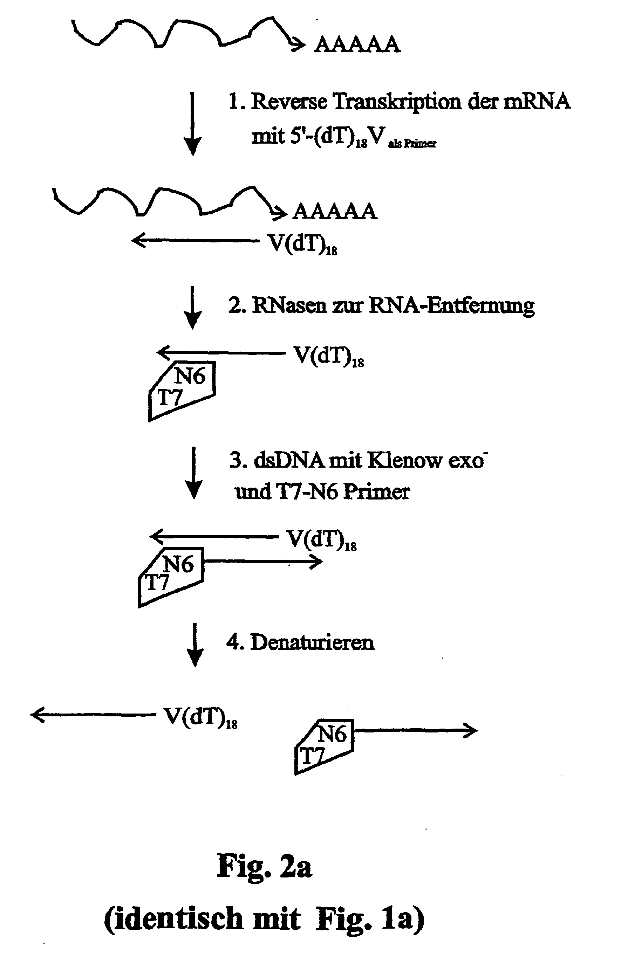

Das erfindungsgemäße Verfahren wird in Fig.1 beispielhaft dargestellt: Zunächst wird ausgehend von RNA die eines DNA-Einzelstranges durch reverse Transkription durchgeführt, wobei ein geankerter Oligo (dT)₁₈V-Primer verwendet wird. Dieses Vorgehen ermöglicht es, den Übergang der Poly(A)-Enden der mRNA zum 3'-UTR-Bereich umzuschreiben. Im nächsten Schritt wird die RNA aus dem RNA/cDNA-Fieteroduplex mittels RNaseH eliminiert und die restliche RNA (ribosomale RNA) durch RNaseI entfernt.

Die Synthese des zweiten, komplementären DNA-Stranges wird zur Einführung der T7-Promotorsequenz durch Verwendung eines speziellen Primers genutzt. Der Primer besteht aus einem Teilabschnitt, in dem 6 beliebige Nukleotide aneinander gereiht wurden, und einem zweiten Teilabschnitt, in der die Sequenz des T7-Polymerase-Promotors umfaßt. Alternativ dazu kann hier der Primer mit der in SEQ ID NO:1 gezeigten Sequenz eingesetzt werden.

Nach Anlagerung des Primers erfolgt die Auffüllung zum Doppelstrang mittels der dem Klenow-Fragment der DNA-Polymerase. Nach Hitzedenaturierung des Doppelstranges wird die Temperatur abgesenkt, wodurch der erfindungsgemäße Primer erneut hybridisieren kann. Ein weiterer DNA-Strang wird durch Elongation des Primers erhalten. Anschließend werden die im Überschuß vorliegenden Primer sowie Primer-verursachte Artefakte (z.B. Dimere) entfernt und die Amplifikation der RNA erfolgt per in vitro-Transkription ausgehend von dem T7-Promotor.

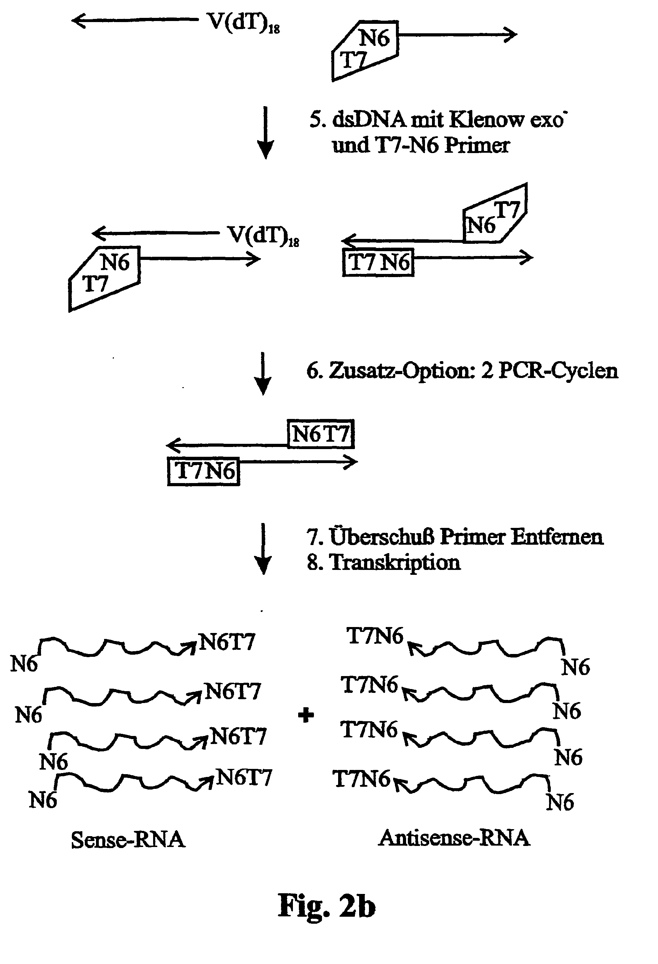

Eine Alternative zu dem obigen Verfahren wird in Fig. 2 beschrieben, bei der die DNA-Doppelstränge vor der Transkription mittels PCR vermehrt werden. Wie in Fig. 2 gezeigt, ermöglicht diese Verfahrensalternative die Erzeugung von Ribonukleinsäuren mit derselben Sequenz wie das Ausgangsmaterials und gleichzeitig die Erzeugung von Ribonukleinsäuren mit der komplementären Sequenz.

Die Abfolge und detailierte Durchführung der Reaktionsschritte des erfindungsgemäßen Verfahrens werden nachfolgend beispielhaft dargestellt:

### 1. Reverse Transkription von 100 ng Gesamt-RNA mit Oligo(dT)₁₈V-Primer:

| **IErststrang-DNA-Synthese:** | |
|---|---|
| RNA (50 ng/µl): | 2 µl |
| Oligo(dT)₁₈ V (5pmol/µl: | 1 µl |
| dNTP-Mix (10 mM): | 0,5 µl |
| DEPC-H₂O | 2 µl |

4 Min. bei 65°C in einem Thermocycler mit Heizdeckel inkubieren dann auf Eis stellen.

| Erststrang-cDNA-Synthesemix | |
|---|---|
| 5 x RT-Puffer | 2 µl |
| 100 mM DTT | 1 µl |
| Rnase-Inhibitor (20 U/µl) | 1 µl |
| Superscript II (200 U/µl) | 0,5 µl |

Den Erststrang-cDNA-Synthesemix auf Eis zusammen pipettieren und zu den Proben im Eisbad geben. Die Proben anschließend in den auf 42°C vorgewärmten Thermocycler stellen.

**Inkubation der Proben:**
42°C/50 Minuten
45°C/10 Minuten
50°C/10 Minuten
70°C/15 Minuten (zur Inaktivierung des Enzymes)

Anschließend Proben auf Eis stellen.

### 2. Entfernung der RNA

| Entfernung der RNA aus dem Reaktionsansatz | |
|---|---|
| Erststrang-cDNA | 10 µl |
| Rnase-Mix (RNase H/Rnase I je 5 U/µl) | 1 µl |

Bei 37°C für 20 Minuten inkubieren, anschließend auf Eis stellen. Zur Entfernung der ribosomalen RNA wurde auf die Verwendung von Rnase verzichtet, da dieses Enzym nur sehr schwer zu aktivieren ist. Die verwendete Rnase I kann an dieser Stelle optimal durch Inkubation bei 70°C für 15 Minuten vollständig inaktiviert werden.

### 3. Rnadom-Hin-und Zurück-priming der Erststrang-cDPIA mit T7-random-Primer

| Random Priming der Erststrang cDNA T7-random Primer | |
|---|---|
| Erststrang-cDNA | 10 µl |
| dNTP-Mix (10 mM) | 0,5 µl |
| artus 6 (T7-random-Primer, 10 pmol/µl) | 3 µl |
| 10x Klenow Puffer | 5 µl |
| H₂O | 30,5 µl |

Inkubation:
Hin-priming:
65°C/1 Minute
37°C 2 Minuten
1 µl Klenow exo-(5U/µl) zur Probe geben
37°C/20 Minuten

Zurück-priming
95°C/1 Minute
37°C/2 Minuten
1 µl Klenow exo-(5 U/µl) zur Probe geben.
37°C/20 Minuten
65°C/15 Minuten (zur Inaktivierung des Enzymes)

### 4. Reinigung der cDNA mit High-Pure PCR Purification Kit (Roche)

| **Reinigung der cDNA:** | |
|---|---|
| Klenow-Reaktionsmix | 50µl |
| Binding-buffer | 250 µl |
| Carrier (cot-1-DNA, 100ng/µl) | 3 µl |

Den Mix auf die Säulchen pipettieren und für 1 Minute in einer Tischzentrifuge bei max. Umdrehung zentrifugieren. Den Durchlauf verwerfen, die Säulchen mit 500 µl Waschpuffer auffüllen und wie oben zentrifugieren. Den Durchlauf verwerfen, die Säulchen mit 200 µl Waschpuffer auffüllen und wie oben zentrifugieren.

Die Säulchen in ein neues 1,5 ml-Eppendorf-Gefäß überführen und mit 50 µl Elutions-Puffer auffüllen, eine Minute inkubieren und dann wie oben zentrifugieren. Den Elutionsvorgang einmal wie beschrieben wiederholen.

### 5. Ethanol-Fällung der gereinigten cDNA

Die Pellet Paint™-Carrier-Stocklösung nicht vortexen und immer dunkel aufbewahren. Längere Lagerung bei -20°C, kleinere Aliquots können für ca. 1 Monat bei 4°C gelagert werden.

| **Ethanol-Fällung:** | |
|---|---|
| Eluat | 100µl |
| Carrier (Pellet Paint^{™}) | 2 µl |
| Natrium-Acetat | 10 µl |
| Alkohol abs | 220 µl |

Den Ansatz gut mischen (nicht vortexen) und die cDNA bei Raumtemperatur für 10 Minuten bei maximaler Umdrehung pelletieren. Den Überstand abnehmen und das Pellet einmal mit 200 µl 70% Ethanol waschen. 1 Minute wie oben zentrifugieren und den Überstand vollständig mit einer Pipette entfernen. Zum Trocknen des Pellets das Eppendorf-Gefäß für ca. 5 Minuten geöffnet bei Raumtemperatur stehen lassen. Nicht in der speedvac trocknen! Anschließend das Pellet in 8 µl Tris-Puffer (pH 8,5) lösen und auf Eis stellen.

### 6. Amplifikation per in vitro-Transkription

| **In vitro Transkription:** | |
|---|---|
| cDNA | 8 µl |
| UTP (75 mM) | 2 µl |
| ATP (75 mM) | 2 µl |
| CTP (75 mM) | 2 µl |
| GTP (75 mM) | 2 µl |
| 10x Puffer | 2 µl |
| T7-RNA-Polymerase | 2 µl |

Alle Reaktionskomponenten auftauen und den Ansatz bei Raumtemperatur, niemals auf Eis zusammenpipettieren, da der Spermidin-Anteil im Reaktionspuffer zu einer Präzipitation des Templates führen würde. Für die Reaktion 0,5 ml bzw. 0,2 ml Rnase-frei PCR-Tubes verwenden.

Die Transkription über Nacht bei 37°C in einem Thermocycler mit Heizdeckel oder in einem Hybridisierungsofen inkubieren. 1-2 µl des Ansatzes auf ein natives 1,5%-Agarose-Gel auftragen. Anschließend 1 µl DNase pro Reaktion zugeben und für weitere 15 Minuten bei 37°C inkubieren. Zur Reinigung der RNA das Rneasy-Kit von Qiagen benutzen und nach dem Protokoll zur RNA-clean-up vorgeben. Die RNA anschließend mit 2x50 µl DEPC-Wasser eluieren und wie bei 6 beschrieben Etanol fällen. Das RNA-Pellet in 5 µl DEPC-Wasser lösen.

Die RNA ist jetzt fertig zur Markierung für die Microarray-Hybridisierung.

## Patentansprüche

1. Verfahren zur Vermehrung von Ribonukleinsäuren, Schritte umfassend, bei denen man
(a) mittels eines Einzelstrang-Primers, einer RNA-abhängigen DNA-Polymerase und Desoxyribonukleotid-Monomeren einen DNA-Einzelstrang durch reverse Transkription aus einer RNA erzeugt;
(b) die RNA entfernt;
(c) mittels eines Einzelstrang-Primers, der die Sequenz eines Promotors und eine beliebige Sequenz von nicht mehr als 6 Nukleotiden umfaßt, einer DNA-Polymerase und Desoxyribonukleotid-Monomeren einen DNA-Doppelstrang erzeugt, wobei die Promotor-Sequenz die Bindung einer RNA-Polymerase ermöglicht und die Synthese eines RNA-Stranges initiiert;
(d) den Doppelstrang in Einzelstränge auftrennt;
(e) mittels eines Einzelstrang-Primers, der die Sequenz eines Promotors und eine beliebige Sequenz von nicht mehr als 6 Nukleotiden umfaßt, einer DNA-Polymerase und Desoxyribonukleotid-Monomeren DNA-Doppelstränge aus den in (d) entstandenen Einzelsträngen erzeugt, wobei die Promotor-Sequenz die Bindung einer RNA-Polymerase ermöglicht und die Synthese eines RNA-Stranges initiiert;
(f) mittels einer RNA-Polymerase und Ribonukleotid-Monomeren eine Vielzahl von RNA-Einzelsträngen erzeugt.

2. Verfahren gemäß Anspruch 1, bei dem RNA-Einzelstränge dieselbe Sinnrichtung (Sequenz) wie das RNA-Ausgangsmaterial aufweisen.

3. Verfahren gemäß Anspruch 1 oder 2, bei dem der in (a) verwendete Einzelstrang-Primer eine Oligo-dT-Sequenz umfaßt.

4. Verfahren gemäß Anspruch 3, bei dem man in (a) einen 5'-(dT)₁₈V-Primer für die reverse Transkription verwendet, worin V ein Desoxyribonukleotid-Monomer bezeichnet, das nicht dT ist.

5. Verfahren gemäß einem der vorstehenden Ansprüche, bei dem man die RNA in (b) durch RNase hydrolysiert.

6. Verfahren gemäß Anspruch 5, bei dem man die RNA in (b) durch RNase I und/oder RNase H entfernt.

7. Verfahren gemäß einem der vorstehenden Ansprüche, bei dem man in (c) einen Einzelstrang-Primer verwendet, der die Sequenz des T7-, T3- oder SP6-RNA-Polymerase-Promotors umfaßt.

8. Verfahren gemäß einem der vorstehenden Ansprüche, bei dem man in (c) einen Einzelstrang-Primer mit einer Gesamtlänge von nicht mehr als 35, vorzugsweise nicht mehr als 30 Nukleotiden verwendet.

9. Verfahren gemäß einem der vorstehenden Ansprüche, bei dem man in (c) einen Einzelstrang-Primer mit der in SEQ ID NO:1 gezeigten Sequenz verwendet.

10. Verfahren gemäß einem der vorstehenden Ansprüche, bei dem man als DNA-Polymerase das Klenow-Fragment der DNA-Polymerase verwendet.

11. Verfahren gemäß Anspruch 10, bei dem man als DNA-Polymerase die Klenow-exo⁻ DNA-Polymerase verwendet.

12. Verfahren gemäß einem der vorstehenden Ansprüche, bei dem man als Desoxyribonukleotid-Monomere dATP, dCTP, dGTP und dTTP einsetzt.

13. Verfahren gemäß einem der vorstehenden Ansprüche, bei dem man in die DNA-Doppelstränge in (d) durch Hitzeeinwirkung in Einzelstränge auftrennt.

14. Verfahren gemäß einem der vorstehenden Ansprüche, bei dem der in (e) eingesetzte Einzelstrang-Primer identisch zu dem in (c) eingesetzten Einzelstrang-Primer ist.

15. Verfahren gemäß einem der vorstehenden Ansprüche, bei dem sich der in (e) eingesetzte Einzelstrang-Primer von dem in (c) eingesetzten Einzelstrang-Primer unterscheidet.

16. Verfahren gemäß einem der vorstehenden Ansprüche, bei dem die in (f) eingesetzte RNA-Polymerase die T7-RNA-Polymerase ist.

17. Verfahren gemäß Anspruch 16, bei dem man vor Zugabe der T7-RNA-Polymerase überschüssige Primer und/oder Primer-verursachte Artefakte entfernt.

18. Verfahren gemäß einem der vorstehenden Ansprüche, bei dem man als Ribonukleotid-Monomere ATP, CTP, GTP und UTP einsetzt.

19. Verfahren gemäß einem der vorstehenden Ansprüche, bei dem eine Vermehrung der Ausgangs-RNA-Sequenz um einen Faktor von mindestens 500, vorzugsweise mindestens 3000 erfolgt.

20. Verfahren gemäß einem der vorstehenden Ansprüche, bei dem man in
(a) ein 5'-(dT)₁₈V-Primer für die reverse Transkription verwendet; und in
(b) RNase zur Aufspaltung der DNA-RNA-Hybride verwendet; und in
(c) und (e) ein Primer mit der SEQ ID NO:1 und die Klenow-exo⁻ DNA-Polymerase verwendet; und in
(d) Hitzeeinwirkung zur Auftrennung der Doppelstränge verwendet; und in
(f) zunächst überschüssige Primer und/oder Primer-verursachte Artefakte entfernt und die T7-RNA-Polymerase verwendet.

21. Verfahren gemäß einem der vorstehenden Ansprüche, bei dem man die in (e) erzeugten DNA-Doppelstränge in Einzelstränge auftrennt und unter Verwendung von mindestens einem Einzelstrang-Primer, einer DNA-Polymerase und den Desoxyribonukleotid-Monomeren zu den DNA-Einzelsträngen jeweils komplementäre DNA-Stränge erzeugt.

22. Verfahren gemäß Anspruch 21, bei dem man die Auftrennung der DNA-Doppelstränge, Primer-Anlagerung und -Elongation mindestens einmal, vorzugsweise mindestens 2 oder 5 mal wiederholt.

23. Verfahren gemäß Anspruch 21 oder 22, bei dem die Auftrennung der DNA-Doppelstränge durch Hitzeeinwirkung erfolgt.

24. Verfahren gemäß einem der Ansprüche 21 bis 23, bei dem man für die Erzeugung weiterer DNA-Doppelstränge Einzelstrang-Primer verwendet, welche die Sequenz des in (c) und/oder (e) verwendeten Primers aufweist.

25. Verfahren gemäß Anspruch 24, bei dem der Einzelstrang-Primer die in SEQ ID NO:1 gezeigte Sequenz aufweist.

26. Verfahren gemäß einem der Ansprüche 21 bis 25, bei dem Ribonukleinsäuren mit derselben Sinnrichtung (Sequenz) wie das RNA-Ausgangsmaterial und Ribonukleinsäuren der komplementären Sequenz gleichzeitig erzeugt werden.

27. Kit zur Vermehrung von Ribonukleinsäuren gemäß einem Verfahren der Ansprüche 1 bis 26, welches die folgenden Bestandteile umfasst:
(a) mindestens einen Einzelstrang-Primer, der die Sequenz eines Promotors und eine beliebige Sequenz von nicht mehr als 6 Nukleotiden umfaßt, wobei die Promotor-Sequenz die Bindung einer RNA-Polymerase ermöglicht und die Synthese eines RNA-Stranges initiiert;
(b) eine RNA-abhängige DNA-Polymerase;
(c) Desoxyribonukleotid-Monomere;
(d) eine DNA-abhängige DNA-Polymerase;
(e) eine RNA-Polymerase; und
(f) Ribonukleotid-Monomere.

28. Kit gemäß Anspruch 27, das zwei verschiedene Einzelstrang-Primer umfaßt.

29. Kit gemäß Anspruch 28, in dem ein Einzelstrang-Primer eine Oligo-dT-Sequenz umfaßt.

30. Kit gemäß Anspruch 29, in dem ein Einzelstrang-Primer eine 5'-(dT)₁₈V-Primer-Sequenz umfaßt, worin V ein Desoxyribonukleotid-Monomer bezeichnet, das nicht dT ist.

31. Kit gemäß einem der Ansprüche 27 bis 30, das zusätzlich RNase I und/oder RNase H umfaßt.

32. Kit gemäß einem der Ansprüche 27 bis 31, in dem ein Einzelstrang-Primer die Sequenz des T7, T3, oder SP6 RNA-Polymerase-Promotors umfaßt.

33. Kit gemäß einem der Ansprüche 27 bis 32, das einen Einzelstrang-Primer der in SEQ ID NO:1 gezeigten Sequenz umfaßt.

34. Kit gemäß einem der Ansprüche 27 bis 33, welches das Klenow-Fragment der DNA-Polymerase umfaßt.

35. Kit gemäß Anspruch 34, welches die Klenow-exo⁻ DNA-Polymerase umfaßt.

36. Kit gemäß einem der Ansprüche 27 bis 35, welches die T7-RNA-Polymerase umfaßt.

37. Kit gemäß einem der Ansprüche 27 bis 36, das eine Zusammenstellung von Reagentien zur Markierung und zum Nachweis von Nukleinsäuren umfaßt.

38. Kit gemäß einem der Ansprüche 27 bis 37, die folgenden Bestandteile umfassend:
(a) einen 5'-(dT)₁₈V-Primer für die reverse Transkription;
(b) RNase;
(c) einen Primer mit der in SEQ ID NO:1 gezeigten Sequenz;
(d) Klenow-exo⁻ DNA-Polymerase;
(e) T7-RNA-Polymerase.

39. Kit gemäß einem der Ansprüche 27 bis 38, das einen Microarray umfaßt.

40. Verfahren zur Analyse von Nukleinsäuren, bei dem man eine Ribonukleinsäure gewinnt, mittels eines Verfahrens gemäß einem der Ansprüche 1 bis 26 vermehrt und unter Verwendung eines Microarrays analysiert.

41. Verfahren gemäß Anspruch 40, bei dem man die Ribonukleinsäure durch Isolierung aus einer biologischen Probe gewinnt.

42. Verfahren gemäß Anspruch 40 oder 41, bei dem man die Ribonukleinsäure vermehrt, durch reverse Transkription in cDNA überführt und die cDNA mittels Microarrays analysiert.

43. Verfahren gemäß einem der Ansprüche 41 bis 42, bei dem man die Menge und oder Sequenz der cDNA analysiert.

44. Primer, der die in SEQ ID NO:1 gezeigte Sequenz aufweist.

## Claims

1. Method for multiplying ribonucleic acids, comprising:
(a) generating a DNA single strand from an RNA by reverse transcription using a single-stranded primer, an RNA-dependent DNA polymerase and deoxyribonucleotide monomers;
(b) removing the RNA;
(c) generating a DNA double strand using a single-stranded primer that comprises the sequence of a promoter and any sequence of not more than 6 nucleotides, a DNA polymerase and deoxyribonucleotide monomers, wherein the promoter sequence allows for the binding of an RNA polymerase and initiates synthesis of an RNA strand;
(d) separating the double strand in single strands;
(e) generating DNA double strands from the single strands generated in (d) using a single-stranded primer that comprises the sequence of a promoter and any sequence of not more than 6 nucleotides, a DNA polymerase and deoxyribonucleotide monomers, wherein the promoter sequence allows for the binding of an RNA polymerase and initiates synthesis of an RNA strand;
(f) generating a plurality of RNA single strands using an RNA polymerase and ribonucleotide monomers.

2. Method according to claim 1, wherein the RNA single strands have the same sense orientation (sequence) as the RNA starting material.

3. Method according to claim 1 or 2, wherein the single-stranded primer used in (a) comprises an oligo-dT sequence.

4. Method according to claim 3, wherein a 5'-(dT)₁₈V primer is used in (a) for reverse transcription, wherein V designates a deoxyribonucleotide monomer other than dT.

5. Method according to any of the preceding claims, wherein in (b) the RNA is hydrolyzed by RNase.

6. Method according to claim 5, wherein in (b) the RNA is removed by RNase I and/or RNase H.

7. Method according to any of the preceding claims, wherein in (c) a single-stranded primer is used, which comprises the sequence of the T7, T3 or SP6 RNA polymerase promoter.

8. Method according to any of the preceding claims, wherein in (c) a single-stranded primer with a total length of not more than 35, preferably not more than 30 nucleotides is used.

9. Method according to any of the preceding claims, wherein in (c) a single-stranded primer having the sequence shown in SEQ ID NO:1 is used.

10. Method according to any of the preceding claims, wherein the Klenow fragment of the DNA polymerase is used as DNA polymerase.

11. Method according to claim 10, wherein the Klenow exo⁻ DNA polymerase is used as DNA polymerase.

12. Method according to any of the preceding claims, wherein dATP, dCTP, dGTP and dTTP are used as deoxyribonucleotide monomers.

13. Method according to any of the preceding claims, wherein in (d) the DNA double strands are separated into single strands by heating.

14. Method according to any of the preceding claims, wherein the single-stranded primer used in (e) is identical to the single-stranded primer used in (c).

15. Method according to any of the preceding claims, wherein the single-stranded primer used in (e) differs from the single-stranded primer used in (c).

16. Method according to any of the preceding claims, wherein the RNA polymerase used in (f) is the T7 RNA polymerase.

17. Method according to claim 16, wherein excessive primer and/or primer-caused artefacts are removed prior to addition of T7 RNA polymerase.

18. Method according to any of the preceding claims, wherein ATP, CTP, GTP or UTP are used as ribonucleotide monomers.

19. Method according to any of the preceding claims, wherein multiplication of the starting RNA sequence by a factor of at least 500, preferably of at least 3000 is achieved.

20. Method according to any of the preceding claims, wherein in
(a) a 5'-(dT)₁₈V primer is used for reverse transcription; and in
(b) RNase is used for the separation of the DNA-RNA hybrids; and in
(c) and (e) a primer having the sequence of SEQ ID NO:1 and the Klenow-exo⁻ DNA polymerase are used; and in
(d) heating is used for the separation of the double strands; and in
(f) excessive primer and/or primer-caused artefacts are initially removed and T7 RNA polymerase is used.

21. Method according to any of the preceding claims, wherein the DNA double strands generated in (e) are separated in single strands, and the respective complementary DNA strands to said DNA single strands are generated using at least one single-stranded primer, a DNA polymerase and the deoxyribonucleotide monomers.

22. Method according to claim 21, wherein the separation of the DNA double strands, primer annealing and elongation are repeated at least one time, preferably at least two or five times.

23. Method according to claim 21 or 22, wherein the separation of the DNA double strands is performed by heating.

24. Method according to any of the claims 21 to 23, wherein single-stranded primers having the sequence of the primer used in (c) and/or (e) are used for generating further DNA double strands.

25. Method according to claim 24, wherein the single-stranded primer has the sequence shown in SEQ ID NO:1.

26. Method according to any of the claims 21 to 25, wherein ribonucleic acids having the same sense orientation (sequence) as the RNA starting material and ribonucleic acids of the complementary sequence are generated at the same time.

27. Kit for multiplying ribonucleic acids according to a method of claims 1 to 26, comprising the following components:
(a) at least one single-stranded primer comprising the sequence of a promoter and any sequence of not more than 6 nucleotides, wherein the promoter sequence allows for the binding of an RNA polymerase and initiates synthesis of an RNA strand;
(b) an RNA-dependent DNA polymerase;
(c) deoxyribonucleotide monomers;
(d) a DNA-dependent DNA polymerase;
(e) an RNA polymerase; and
(f) ribonucleotide monomers.

28. Kit according to claim 27, comprising two different single-stranded primers.

29. Kit according to claim 28, wherein the single-stranded primer comprises an oligo-dT sequence.

30. Kit according to claim 29, wherein the single-stranded primer comprises a 5'-(dT)₁₈V primer sequence, wherein V designates a deoxyribonucleotide monomer other than dT.

31. Kit according to any of claims 27 to 30, further comprising RNase I and/or RNase H.

32. Kit according to any of claims 27 to 31, wherein a single-stranded primer comprises the sequence of the T7, T3 or SP6 RNA polymerase promoter.

33. Kit according to any of claims 27 to 32, comprising a single-stranded primer having the sequence shown in SEQ ID NO:1.

34. Kit according to any of claims 27 to 33, comprising the Klenow fragment of DNA polymerase.

35. Kit according to claim 34, comprising the Klenow-exo⁻ DNA polymerase.

36. Kit according to any of claims 27 to 35, comprising the T7 RNA polymerase.

37. Kit according to any of claims 27 to 36, comprising a combination of reagents for labeling and detecting nucleic acids.

38. Kit according to any of claims 27 to 37, comprising the following components:
(a) a 5'-(dT)₁₈V primer for reverse transcription;
(b) RNase;
(c) a primer having the sequence shown in SEQ ID NO:1;
(d) Klenow-exo⁻ DNA polymerase;
(e) T7 RNA polymerase.

39. Kit according to any of claims 27 to 38, comprising a microarray.

40. Method for analyzing nucleic acids, wherein a ribonucleic acid is obtained, multiplied by a method according to any of claims 1 to 26, and analyzed by use of a microarray.

41. Method according to claim 40, wherein the ribonucleic acid is obtained by isolation from a biological sample.

42. Method according to claim 40 or 41, wherein the ribonucleic acid is multiplied, converted into cDNA by reverse transcription, and the cDNA is analyzed by use of a microarray.

43. Method according to any of claims 41 to 42, wherein the amount or the sequence of the cDNA is analyzed.

44. Primer having the sequence shown in SEQ ID NO:1.

## Revendications

1. Procédé pour l'amplification des acides ribonucléiques, comportant des étapes consistant à :
a) produire, au moyen d'une amorce simple brin, d'une ADN-polymérase ARN-dépendante et de monomères désoxyribonucléotidiques, un simple brin d'ADN par transcription inverse à partir d'un ARN ;
b) éliminer l'ARN :
c) produire un double brin d'ADN au moyen d'une amorce simple brin comprenant la séquence d'un promoteur et une séquence désirée de 6 nucléotides au maximum, d'une ADN-polymérase et de monomères désoxyribonucléotidiques, la séquence promoteur permettant la liaison d'une ARN-polymérase et initiant la synthèse d'un brin d'ARN ;
d) séparer le double brin en simples brins ;
e) produire des doubles brins d'ADN à partir des simples brins formés dans (d) au moyen d'une amorce simple brin comprenant la séquence d'un promoteur et une séquence désirée de 6 nucléotides au maximum, d'une ADN-polymérase et de monomères désoxyribonulcléotidiques, la séquence promoteur permettant la liaison d'une ARN-polymérase et initiant la synthèse d'un brin d'ARN ;
f) produire une multitude de simples brins d'ARN au moyen d'une ARN-polymérase et de monomères ribonucléotidiques.

2. Procédé selon la revendication 1, dans lequel les simples brins d'ARN présentent la même orientation (séquence) que le matériel ARN initial.

3. Procédé selon la revendication 1 ou 2, dans lequel l'amorce simple brin utilisée dans l'étape (a) comprend une séquence oligo-dT.

4. Procédé selon la revendication 3, dans lequel on utilise une amorce 5'-(dT)₁₈V dans l'étape (a) pour la transcription inverse, dans laquelle V représente un monomère désosyribonucléotidique qui n'est pas dT.

5. Procédé selon l'une des revendications précédentes, dans lequel dans l'étape (b) on hydrolyse l'ARN au moyen d'une RNase.

6. Procédé selon la revendication 5, dans lequel dans l'étape (b) on élimine l'ARN moyen de la RNase I et/ou de la de la RNase H.

7. Procédé selon l'une des revendications précédentes, dans lequel on utilise dans l'étape (c) une amorce simple brin qui comprend la séquence du promoteur de l'ARN-polymérase 7, T3 ou SP6.

8. Procédé selon l'une des revendications précédentes, dans lequel on utilise dans l'étape (c) une amorce simple brin d'une longueur totale n'excédant pas 35 nucléotides, de préférence n'excédant pas 30 nucléotides.

9. Procédé selon l'une des revendications précédentes, dans lequel on utilise dans l'étape (c) une amorce simple brin avec la séquence indiquée dans SEQ ID NO:1.

10. Procédé selon l'une des revendications précédentes, dans lequel on utilise comme ADN-polymérase le fragment de Klenow de l'ADN-polymérase.

11. Procédé selon la revendication 10, dans lequel on utilise comme ADN-polymérase l'ADN-polymérase Klenow-exo⁻.

12. Procédé selon l'une des revendications précédentes, dans lequel on utilise comme monomères désoxyribonucléotidiques le dATP, le dCTP, le dGTP et le dTTP.

13. Procédé selon l'une des revendications précédentes, dans lequel on sépare les doubles brins d'ADN dans l'étape (d) en simples brins sous l'effet de la chaleur.

14. Procédé selon l'une des revendications précédentes, dans lequel l'amorce simple brin utilisée dans l'étape (e) est identique à l'amorce simple brin utilisée dans l'étape (c).

15. Procédé selon l'une des revendications précédentes, dans lequel l'amorce simple brin utilisée dans l'étape (e) est différente de l'amorce simple brin utilisée dans l'étape (c).

16. Procédé selon l'une des revendications précédentes, dans lequel l'ARN-polymérase utilisée dans l'étape (f) est l'ARN-polymérase T7.

17. Procédé selon la revendication 16, dans lequel on élimine l'amorce en excès et /ou les artéfacts produits par l'amorce avant d'ajouter l'ARN-polymérase T7.

18. Procédé selon l'une des revendications précédentes, dans lequel on utilise comme monomères ribonucléotidiques l'ATP, le CTP, le GTP et l'UTP.

19. Procédé selon l'une des revendications précédentes, dans lequel on effectue une amplification de la séquence ARN initiale d'un facteur d'au moins 500, de préférence d'au moins 3000.

20. Procédé selon l'une des revendications précédentes, dans lequel on utilise dans :
(a) une amorce 5'-(dT)₁₈V pour la transcription inverse ; et dans
(b) une RNase pour la dissociation de l'hybride ADN-ARN ; et dans
(c) et (e) une amorce comprenant la séquence ID NO:1 et une ADN-polymérase Klenow-exo⁻ ; et dans
(d) les effets de la chaleur pour séparer le double brin ; et dans
(f) on élimine d'abord l'amorce en excès et/ou les artéfacts produits par l'amorce avant d'ajouter l'ARN-polymérase T7.

21. Procédé selon l'une des revendications précédentes, dans lequel on sépare l'ADN double brin produit dans l'étape (e) en simples brins et dans lequel on produit en utilisant au moins une amorce simple brin, une ADN-polymérase et des monomères désosyribonucléotidiques des brins d'ADN complémentaires de chacun des simples brins d'ADN.

22. Procédé selon la revendication 21, dans lequel on répète la séparation des doubles brins d'ADN, et la fixation et l'élongation de l'amorce au moins une fois, de préférence 2 ou 5 fois.

23. Procédé selon la revendication 21 ou 22 , dans lequel la séparation des doubles brins d'ADN s'effectue sous l'effet de la chaleur.

24. Procédé selon l'une des revendications 21 à 23, dans lequel on utilise, pour la production d'autres doubles brins d'ADN, une amorce simple brin qui présente la séquence de l'amorce utilisée dans l'étape (c) et/ou l'étape (e).

25. Procédé selon la revendication 24, dans lequel l'amorce simple brin présente la séquence indiquée dans SEQ ID NO:1.

26. Procédé selon l'une des revendications 21 à 25, dans lequel les acides ribonucléiques avec la même orientation (séquence) que le matériel ARN initial et les acides ribonucléiques de la séquence complémentaire sont produits simultanément.

27. Kit pour l'amplification des acides ribonucléiques selon l'un des procédés des revendications 1 à 26 comprenant les éléments suivants :
(a) au moins une amorce simple brin comprenant la séquence d'un promoteur une séquence souhaitée de 6 nucléotides au maximum, la séquence promoteur permettant la liaison d'une ARN-polymérase et initiant la synthèse d'un brin d'ADN ;
(b) une ADN-polymérase ARN dépendante ;
(c) des monomères désoxyribonucléotidiques ;
(d) une ADN-polymérase ADN dépendante ;
(e) une ARN-polymérase ;
(f) des monomères ribonucléotidiques.

28. Kit selon la revendication 27 comprenant deux amorces simple brin différentes.

29. Kit selon la revendication 28, dans lequel une amorce simple brin comprend une séquence oligo-dT.

30. Kit selon la revendication 29, dans lequel une amorce simple brin comprend une séquence 5'-(dT)₁₈V, dans laquelle V représente un monomère désoxyribonucléotidique qui n'est pas dT.

31. Kit selon l'une des revendications 27 à 30, qui comprend également une RNase I et/ou une RNase H.

32. Kit selon l'une des revendications 27 à 31, dans lequel une amorce simple brin comprend la séquence du promoteur de l'ARN-polymérase T7, T3 ou SP6.

33. Kit selon l'une des revendications 27 à 32, qui comprend une amorce simple brin de la séquence indiquée dans SEQ ID NO:1.

34. Kit selon l'une des revendications 27 à 33, qui comprend le fragment de Klenow de l'ADN-polymérase.

35. Kit selon la revendication 34, qui comprend l'ADN-polymérase Klenow-exo⁻.

36. Kit selon l'une des revendications 27 à 35, qui comprend l'ARN-polymérase T7.

37. Kit selon l'une des revendications 27 à 36, qui comprend un ensemble de réactifs pour le marquage et la détection des acides nucléiques.

38. Kit selon l'une des revendications 27 à 37, qui comprend les éléments suivants :
(a) une amorce 5'-(dT)₁₈V pour la transcription inverse ;
(b) une RNase ;
(c) une amorce comprenant la séquence indiquée dans SEQ ID NO:1 ;
(d) l'ADN-polymérase Klenow-exo⁻ ;
(e) l'ARN-polymérase T7.

39. Kit selon l'une des revendications 27 à 38, qui comprend une puce à ADN (microarray).

40. Procédé pour l'analyse des acides nucléiques, dans lequel on obtient un acide ribonucléique que l'on amplifie au moyen d'un procédé selon l'une des revendications 1 à 26 et que l'on analyse en utilisant une puce à ADN.

41. Procédé selon la revendication 40, dans lequel on obtient l'acide ribonucléique par isolation à partir d'un échantillon biologique.

42. Procédé selon la revendication 40 ou 41, dans lequel on amplifie l'acide ribonucléique, on le convertit en ADNc par transcription inverse et on analyse l'ADNc au moyen d'une puce à ADN.

43. Procédé selon l'une des revendications 41 à 42, dans lequel on analyse la quantité et/ou la séquence de l'ADNc.

44. Amorce qui présente la séquence indiquée dans SEQ ID NO:1.
